(19) Europäisches Patentamt / European Patent Office / Office européen des brevets

(11) **EP 4 023 242 A1**

(12) **EUROPEAN PATENT APPLICATION**
published in accordance with Art. 153(4) EPC

(43) Date of publication:
**06.07.2022 Bulletin 2022/27**

(21) Application number: **20860766.3**

(22) Date of filing: **28.08.2020**

(51) International Patent Classification (IPC):
**A61K 38/17** *(2006.01)*          **A61P 13/12** *(2006.01)*

(86) International application number:
**PCT/CN2020/000197**

(87) International publication number:
**WO 2021/042645 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **03.09.2019  CN 201910847029**

(71) Applicant: **Qi, Zhankai**
**Shanghai 200065 (CN)**

(72) Inventors:
• **QI, Hyatt**
**Shanghai 200065 (CN)**
• **QI, Zhankai**
**Shanghai 200065 (CN)**

(74) Representative: **Sun, Yiming**
**HUASUN Patent- und Rechtsanwälte**
**Friedrichstraße 33**
**80801 München (DE)**

(54) **APPLICATION OF COBRA NEUROTOXIN PEPTIDE MOLECULE IN TREATING NEPHRITIS PROTEINURIA**

(57)     Provided are applications of cobra neurotoxin peptide molecules in treating nephritis proteinuria.

**Description**

**FIELD OF THEINVENTION**

The invention relates to a group of cobra neurotoxin monomer molecules which can inhibit adriamycin induced rat nephritis proteinuria and streptozotocin (STZ) - induced rat nephritis proteinuria and improve renal function, belonging to the field of Biochemistry and biopharmaceutical technology.

**BACKGROUND OF THE INVENTION**

Nephropathy is a kind of glomerular diseases with multiple causes, which can be categorized into primary nephritis and secondary nephritis. Primary nephritis may be caused due to immune reaction, pro-inflammatory cytokine due to various bacterial, viral, protozoan infections or by non immune mechanisms. Secondary nephritis may be caused by diabetes, hypertension, Henoch Schonlein purpura, systemic lupus erythematosus, multiple nephritic sac and gouty nephropathy, etc. Regardless of primary or secondary nephropathy, there are a considerable number of patients will develop into chronic nephritis, which is difficult to heal. If the development of nephritis cannot be prevented, renal failure might be inevitable due to no effective therapeutic agents at present.

Traditionally, the biomarkers such as routine urinary protein, blood urea nitrogen bun and blood creatinine Cr are used for the diagnosis of renal function injury, however, when large amount of proteinuria and/or blood urea nitrogen and/or creatinine are detected, very likely kidney has experienced severe and irreversible impairments. Actually, patients with years of nephritis will start to have small amount of blood albumin leaking into the urine, this is the first stage of chronic nephritis, which is called micro albuminuria. Although micro albuminuria could also be associated with cardiovascular related diseases such as hypertension, hyperlipidemia, atherosclerosis, whilst when there are significant increase of other biomarkers of renal function such as immunoglobulin, $\beta$ 2 microglobulin, $\alpha$ 1 microglobulin and transferrin as well, it is confirmed that there some pathological changes at kidney level. [1-11]

When the function glomerular is affected, $\beta$ 2 microglobulin will leak into urine, resulting in a significant increase in its content in the urine, so urine $\beta$ 2 microglobulin can directly reflect glomerular function. [12, 13] $\alpha$1 microglobulin in blood, when passes through glomerular filtration membrane, it will be reabsorbed in the renal proximal convoluted tubules, and only a small amount will be excluded into the urine, so when there is an increased level of $\alpha$ 1 microglobulin content in the urine, it reflects the abnormalities of renal tubular reabsorption function and glomerular filtration function [14, 15, 16]. Blood transferrin is an iron containing protein in plasma, which is mainly responsible for carrying iron. Transferrin molecules have less negative charge and can pass easily through the kidney electronic charge barrier of the ball, especially in the early stage of diabetic nephropathy, due to decreases of the negative charge of the filtration membrane while the pore is still unchanged, transferrin can appear earlier than albumin in the urine and which can be used as a more sensitive biomarker for the diagnosis of early renal disease, when renal disorder happens, its content will increase significantly in urine [17-21]. Immunoglobulin G is a direct indicator of autoimmune response, when tracing amount of albumin, $\beta$ 2 microglobulin, $\alpha$ 1 microglobulin, transferrin, immunoglobulin G appears in human urine, these relevant indicators can directly reflect the human renal function and pathologic changes, when there is lesion in kidney, those indicators will increase significantly. Experimental results prove that simultaneous test of albumin, $\beta$ 2 microglobulin, $\alpha$ 1 microglobulin, transferrin and immunoglobulin G will bring much reliable parameters for clinic diagnosis and can fix the discrepancy of each individual test. [1-3].

In early stage, the clinical symptoms of nephropathy patients are mild which can last for many years, during this period, the kidney filtration is still functioning normally, If microalbuminuria can be diagnosed and treated in time, it is possible to reverse or improve the nephritis; however, if the course of disease progresses freely, more protein will leak into the urine. This stage can be called massive proteinuria stage, the filtration function of the kidney starts to decline with the increase of proteinuria and the human body will retain all kinds of metabolite waste, and blood urea nitrogen (BUN), and/or serum creatinine (CR) will be increasing with the progression of kidney damage, at that time, in addition to the common clinical symptoms such as proteinuria and edema, there is high chance to develop hypertension and anemia, accompanied by different degrees of kidney damage. In this period, patients with nephropathy are about to enter the end-stage. At present, glucocorticoids are the main treatment for nephritis, but with many side effects, including gastrointestinal mucosal bleeding, hypoglycemia, hypotension, risk of infections, osteoporosis, femoral head necrosis ext, which can cause severe problem to muscular skeleton system, and may also cause mental excitement.

The treatment of end-stage nephropathy is mainly through kidney transplantation or dialysis, but the long-term treatment will become a heavy burden for the patients mentally and physically. therefore, early diagnosis and treatment of nephropathy is the key to improve the treatment result and prognosis of nephropathy patients.

Scientists have tried different way to develop medicaments for the treatment of nephropathy, including snake venom products, for example, Chinese cobra venom has been reported for treating kidney disease (CN patent 201210228609, physically modified Chinese cobra venom in the preparation of drugs for the treatment of acute and chronic nephropathy), however there are a wide variety of components in cobra venom such as neurotoxin, cytotoxin, cardiotoxin, nerve growth factor, hemolysin (DLP), CVA protein, membrane active polypeptide, cobra toxic factors and other components, such as alkaline phosphomonoesterase, phosphodiesterase, acetylcholinesterase, L-amino acid oxidase, nuclear enzyme, glyconucleases, proteolytic enzymes, etc. for cobra venom preparations, mixed toxins will pose a great threat to the safety, especially for the treatment of kidney disease, as mixture of snake venom can cause acute renal failure in case of injury by snake bite, severe reaction will be provoked by mixture of snake venom[22, 23, 24]. Synergism is a significant phenomenon present in snake venoms that may be an evolving strategy to potentiate toxicities. A synergism exists between different toxins or toxin complexes in various snake venoms. The predominant toxins, such as PLA2s, three-finger toxins (3FTxs) play essential roles in synergistic processes[25]. Synergism between venom toxins exists for a range of snake species. Two or more venom components interact directly or indirectly to potentiate toxicity to levels above the sum of their individual toxicities. From a molecular perspective, synergism may exist in two general forms:

(1) Intermolecular synergism, when two or more toxins interact with two or more targets on one or more (related) biological pathways, causing synergistically increased toxicity.

(2) Supramolecular synergism, when two or more toxins interact with the same target in a synergistic manner or when two or more toxins associate

and create a complexes with increased toxicity[26]. An example of intermolecular synergism is $\alpha$-neurotoxins from elapid(s) combined with other toxins, causing a synergistic potent toxic effect leading to flaccid paralysis and respiratory failure in victims and prey[26] . In a study about how Mamba snake (Cobra) venom caused high mortality, scientists revealed that potassium channel blocking activity was only one of the mechanisms. There are various other pathways involved. The combined toxicity induced at various organ levels by different dendrotoxins leading to all-around damage was a concern towards high mortality[27].

Study of Strydom and Botes (1970) showed that separated venom fractions from the venom of the related Eastern green mamba (Cobra) were devoid of lethality after 48 hours when administered alone, whereas the whole venom was lethal within 10 minutes when administered at a similar dose [28].

Synergistic toxins are known to enhance the toxicity of certain toxins. Individually, these proteins are less toxic, but they act as potent toxins when injected into mice in combination. These toxins show similarities with that of neurotoxin or cytotoxins in amino acid sequence and number of half. Such synergistically acting toxins are called synergistic toxins[29].

The physically modified Chinese cobra venom may reduce the toxicity of snake venom, but as a mixture, the components are complex and the quality control is difficult, so there is still the possibility of synergy between toxins.

In addition, technicians are trying to treat diabetic nephropathy with unidentified cobra neurotoxin or its mixture (collectively known as cobra neurotoxin). However, these neurotoxins cause safety problems because of their unclear composition, so the most effective way to avoid such serious side effect of synergism is to replace the mixture with monomer molecules.

## DETAILED DESCRIPTION OF THE INVENTION

Adriamycin induced rat nephritis model can show typical nephropathy syndrome. The pathological changes of the animal model are similar to those of human minimal lesion nephropathy and focal segmental glomerulosclerotic nephropathy; [30] Streptozotocin (STZ) induced diabetic nephropathy in 'rats can make rats demonstrate typical diabetic nephropathy symptoms. the pathological changes of the animal models are similar to minor lesions of human diabetic nephropathy. [31]

The present invention, through adriamycin induced nephritis model and streptozotocin (STZ) - induced diabetic nephropathy model recognized by professionals in this field, allows us to observe the therapeutic effect of cobra neurotoxin polypeptide molecule on various kinds of microalbuminuria in diabetic nephropathy and other kinds of nephritis.

According to the publication, it's the first time that we have used cobra neurotoxin mono molecule to study the therapeutic effect of diabetic nephropathy and other nephropathy, in addition , the invention discloses the application of a group of cobra neurotoxin polypeptide molecules in the treatment of nephropathy proteinuria. the group of cobra neurotoxin polypeptide molecules can decrease the level of albumin, immunoglobulins, $\beta$ 2 microglobulin, $\alpha$ 1 microglobulin and transferrin in the urine, which represents the stop of renal auto-immune reaction progression and the improvement of renal pathologic changes.

Another advantage of the invention is that the group of cobra neurotoxin polypeptide monomer molecules can avoid serious side effect caused by the synergism of snake venom mixture or neurotoxin mixture as the clincal manifestation is renal failure when bitten by snake.

Our invention disclosed for the first time that the postsynaptic neurotoxin monomer molecule from Naja atra can inhibit adriamycin induced rat nephritis proteinuria and streptozotocin (STZ) - induced rat nephritis proteinuria; Other postsynaptic neurotoxins from elapidae include Naja kaouthia neurotoxin; Bungarus neurotoxin; King cobra neurotoxin and Black mamba cobra neurotoxin have almost reached the same experimental results in the treatment of rat nephritis proteinuria, as they all have the common three finger protein functional structure and they are all postsynaptic neurotoxins and antagonists of acetylcholine receptors. Acetylcholine receptors are involved in the regulation of immune inflammatory response, [32, 33, 34, 35], which make them have the common therapeutic effect. These neurotoxins contain the following mature proteins or polypeptides with the amino acid sequences (FASTA) as follows:

Postsynaptic neurotoxins from Naja atra (cobrotoxin)

SEQ ID No.1
lechnqqssq tptttgcsgg etncykkrwr dhrgyrterg cgcpsvkngi einccttdrc nn
SEQ ID No.2
mktlllltllv vtivcldlgy tlechnqqss qtptttgcsg getncykkrw rdhrgyrter gcgcpsvkng ieinccttdr cnn
SEQ ID No.3
lechnqqssq tptttgcsgg etncykkrwr dhrgyrterg cgcpivkngi esnccttdrc nn

Postsynaptic neurotoxins from Bungarus

SEQ ID No.4
ivchttatsp isavtcppge nlcyrkmwcd afcssrgkvv elgcaatcps kkpyeevtcc stdkcnphpk qrpg
SEQ ID No.5
ivchttatip ssavtcppge nlcyrkmwcd afcssrgkvv elgcaatcps kkpyeevtcc stdkcnhppk rqpg

Postsynaptic neurotoxins from King cobra

SEQ ID No.6
ircfitpdvt sqacpdgqni cytktwcdnf cgmrgkrvdl gcaatcptvk pgvdikccst dncnpfptre rs
SEQ ID No.7
ircfitpdvt sqacpdghvc ytkmwcdnfc gmrgkrvdlg caatcpkvkp gvnikccsrd ncnpfptrkr s
SEQ ID No.8
tkcyktgdri iseacppgqd lcymktwcdv fcgtrgrvie lgctatcptv kpheqitccs tdncdphhkm lq

Postsynaptic neurotoxins from Naja kaouthia

SEQ ID No.9
lechnqqssq apttktcsge tncykkwwsd hrgtiiergc gcpkvkpgvn lnccrtdrcn n
SEQ ID No.10
lechnqqsiq tptttgcsgg etncykkrwr dhrgyrterg cgcpsvkngi einccttdrc nn
SEQ ID No.11
mktlllltllv vtivcldlgy tlechnqqss qtptttgcsg getncykkrw rdhrgyrter gcgcpsvkng ieinccttdr cnn

Postsynaptic neurotoxins from Black Mamba

SEQ ID No.12
xicynhqstt rattksceen scykkywrdh rgtiiergcg cpkvkpgvgi hccqsdkcny
SEQ ID No.13
ricynhqstt rattksceen scykkywrdh rgtiiergcg cpkvkpgvgi hccqsdkcny
SEQ ID No.14
rtcnktfsdq skicppgeni cytktwcdaw csrrgkivel gcaatcpkvk agvgikccst dncnlfkfgk pr

Another advantage of the invention is for product manufacturing, because the postsynaptic neurotoxin monomer molecule from elapidae disclosed by the invention has a clear amino acid sequence, it can be produced through genetic engineering, which solves the practical problem of scarcity of snake venom resources; even if we continue to obtain postsynaptic neurotoxin through the separation and purification of natural snake venom, it is easier to control the quality and purity due to the identified amino acid sequence in the process, building up a necessary basis for the drug development of monomer from snake venom.

The above scheme is further described below in combination with specific embodiments. It should be understood that these embodiments are used to illustrate the invention and not to limit the scope of the invention.

## BRIEF DESCRIPTION OF THE DRAWINGS

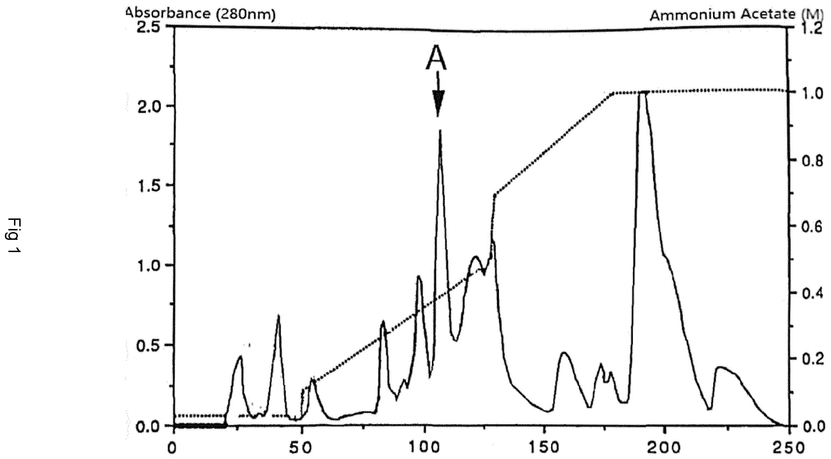

Fig1 is 12 protein peaks of crude venom from Naja atra separated by TSK CM-650 (M) column with ammonium acetate as buffer and eluent

## Examples:

Example A: Obtaining neurotoxin polypeptide SEQ ID No1 from Naja atra

1. Separation and Purification of venom toxins from Naja atra

Dissolve 1g of Naja atra crude venom in 25ml 0.025 mol ph6 0 ammonium acetate buffer, centrifugation at low temperature, and take the supernatant; Use 0.025 mole ph6 0 ammonium acetate solution to balance TSK cm-650 (m) column; After loading the sample, 0.1 ~ 0.5 mol and 0.7 ~ 1.0 mol, pH5. 9 ammonium acetate buffer was used for elution in two compartment gradient, and the UV detection parameter was set at 280nm; Elution flow rate: 48ml / h; Various toxin components were collected according to the recorded spectrum, and 12 protein peaks were washed out from the collected solution.

2. Identify the Naja atra toxin with affinity to nicotinic acetylcholine receptor (nAChR)

The method was based on the affinity test between 12 protein peaks and nicotinic acetylcholine receptor (nAChR).

The principle of affinity test

Because $\alpha$-bungarotoxin has high affinity with the nicotinic acetylcholine receptor (nAChR) , the cobra neurotoxin can compete with $\alpha$-bungarotoxin for binding with nicotinic acetylcholine receptor , and only these $\alpha$-bungarotoxin labeled with the radionuclide 1251 and bound with the nicotinic acetylcholine receptor will be precipitated and be counted by the Gamma immunocount instrument whilst unbound $\alpha$-bungarotoxin will be washed out. Therefore, 12 isolated proteins binding inhibition rate of $\alpha$-bungarotoxin with nicotinic acetylcholine receptor (nAChR) could be used to measure the affinity between the isolated proteins and nicotinic acetylcholine receptor (nAChR), that's each isolated protein's bioactivity, which could be further determined by the $\gamma$ counting value per second (Bq) measured by immune counter, and it becomes the index of proteins bioactivity.

The way of implementation include the method of binding inhibition rate of 1251 radioactive labeling- $\alpha$- bungarotoxin with nicotinic acetylcholine receptor (%) which reflects the activity of each protein.

The method for identifying cobra neurotoxin with high affinity with nicotinic acetylcholine receptor (nAChR) includes the following steps:

Take the above isolated snake venom protein (just one purified of 12 protein peaks each time) and rat skeletal muscle nAChR extract, 1 $\mu$ L monoclonal antibody against acetylcholine nicotine receptor (mab35) 5.9mg/ml, labeled with radionuclide 125l $\alpha$-Bungarotoxin 1 $\mu$ l(125l-n $\alpha$- Btx)0.18 $\mu$ G / ml, after mixing, stand at 4 °C for more than 10 hours; The next day, Rabbit anti rat IgG (4.5mg / ml) was added for 10 $\mu$ L, stand at 4 °C for 2 hours; Centrifuge at 13000 rpm for 5 min, and wash the precipitate with Triton X-100 lotion for 3 times; $\gamma$ count value per second (BQ) of protein activity index was measured by immunocounter; CaBTX, CBSA and C Snake Venoms refer to the BQ values of positive control $\alpha$BTX, negative control BSA and isolated snake venom components respectively.

Calculation of "125I-αBtx-nAChR binding inhibitory rate (%)"

$$= 100 \times (CBSA\text{-}C\ cbx) \div (CBSA\text{-}C\alpha Btx),$$

Where
CBSA means concentration using beef serum albumin to inhibit 125l-$\alpha$Btx-nAChR binding, 0% inhibited.
CaBtx means concentration using $\alpha$-bungarotoxin to inhibit 125l-$\alpha$Btx-nAChR binding, 100% inhibited.
Ccbx means concentration using isolated snake venom (cobrotoxin ) to inhibit 1251-$\alpha$Btx-nAChR binding.

The results showed that peak A was the active peak, the inhibition rate was about 50%, indicating that it had neurotoxin activity and higher affinity with nAChR. (Figure 1)

3. The primary structure analysis of peak A protein - amino acid sequencing

The amino acid sequencing method comprises the following steps:
Reversed phase high performance liquid chromatography (RP-HPLC) column (4.6 × 250mm, VYDAC RP-C8, 5 μ m) was used for purification and desalt of peak A; the N-terminal and C-terminal amino acid sequences were determined by ABI 491 protein sequence analyzer; The N-terminal amino acids obtained by sequencing were analyzed by BLSAT, and the theoretical amino acid sequence of cobra neurotoxin was predicted by comparing with the existing cobra neurotoxin sequence ; the peptide coverage of cobra neurotoxin was analyzed. The experimental method was to enzymolysis the protein samples with trypsin, chymotrypsin and Glu-C enzyme respectively; Then, LC-MS / MS (xevog2 XS QTOF waters) was used to analyze the peptide samples after enzymatic hydrolysis; Use Unifi (1.8.2, waters) software to analyze LC-MS / MS data, and determine the peptide coverage of the test article according to the algorithm results. Finally, the sequence was confirmed by Edman degradation method.
The amino acid sequence of the primary structure of peak A protein obtained by sequencing is: (see amino acid sequence list < 400 > 1), and the corresponding amino acid sequence FASTA form is: (SEQ ID No.1). Cobra neurotoxin polypeptide (SEQ ID No.2-SEQ ID No.14) can be obtained by the same method.

Example B: Use cobra neurotoxin polypeptides SEQ ID No.1 and SEQ ID No.2 for the treatment of adriamycin induced nephritis proteinuria in rats

1 . Experimental animals and groups

80 male SD rats weighing 160-180g were randomly divided into 2 groups: 20 in the control group and 60 in the model group. 40 rats in model group, after success in modeling, were randomly divided into cobra neurotoxin polypeptide treatment group and model group.
Each group of the three, namely control group, treatment group and model group were randomly divided into two groups again, with 10 rats in each group to test cobra neurotoxin peptide SEQ ID No. 1 and SEQ ID No. 2, the remaining rats were out of the groups.

2 . Modeling method

Glomerulosclerotic rats were established by unilateral nephrectomy combined with tail vein injection of adriamycin twice every 2 weeks. Model. [30] rats rested for 3 days after unilateral nephrectomy, and then injected adriamycin 3mg / kg and adriamycin 2mg/kg from caudal vein on the 4th and 18th days respectively, the urinary protein of the surviving rats was detected 7 days after the second injection of adriamycin, the quantitative urinary protein over 100mg / 24h suggesting the success modeling, then they were randomly divided into model group or treatment group and under observation continuously for 8 weeks. The control group was injected into caudal vein on the 4th and 18th day respectively with normal saline 0.5ml.

During the 8 weeks of observation, the treatment group was given cobra neurotoxin polypeptide · 20ug / kg by gavage once a day, continuously for 8 weeks; the model group and the control group were perfused with normal saline once a day for 8 weeks.

3. Collection and detection of urinary protein

After the last treatment, the rats were placed in the metabolic cage to collect 10ml of urine, centrifuged at 3500r / min for 10min, the supernatant was extracted into the cryopreservation tube, stored in the refrigerator at - 80 °C for standby, and the urinary A1 microglobulin was detected by enzyme-linked immunosorbent assay, β 2-microglobulin, microalbumin, transferrin and immunoglobulin G (IgG), operate according to the instructions of ELISA kit.
Anesthetized rats were injected intraperitoneally with 1.5% Pentobarbital Sodium (50mg / kg). After the corneal reflex disappeared, the abdominal cavity was opened and the abdominal aorta was exposed. The abdominal aorta collected 10ml of whole blood and placed in a centrifuge tube. After standing for 2h, centrifuged at 3500r / min for 10min. The supernatant was extracted in a cryopreservation tube and stored in a refrigerator at - 80 °C for standby. The serum creatinine, urea nitrogen, cholesterol and triglyceride were detected by a full-automatic biochemical analyzer.

4. Comparison of urinary protein content between control group, modeling group and treatment group

TABLE-1 SEQ ID No.1 cobrotoxin polypeptide was used for treatment group

Table-1

| adriamycin 3+2mg/kg | UALB ($\mu$g/ml) | a1-MG ($\mu$g/ml) | B2-MG (ng/ml) | TRF ($\mu$g/ml) | IgG ($\mu$g/ml) |
| --- | --- | --- | --- | --- | --- |
| control group | 0.41 ±0.08 | 0.29 ±0.06 | 8.26 ±1.77 | 0.04 ±0.02 | 0.09 ±0.04 |
| modeling group | 1.26 ±0.23** | 0.61 ±0.17** | 40.12 ±11.28* | 0.24 ±0.07* | 0.47 ±0.16* |
| cobrotoxin group | 0.89 ±0.08 | 0.42 ±0.08 | 29.29 ±6.93 | 0.17 ±0.08 | 0.34 ±0.08 |

a) The effect of cobrotoxin polypeptide on the microalbuminuria (UALB) of adriamycin induced diabetic nephropathy in rats, compared with the control group, the microalbuminuria (UALB) of the modeling group was significantly increased; compared with the modeling group, the microalbuminuria of cobrotoxin polypeptide group was significantly lower. * * It indicates that the cobrotoxin polypeptide group is compared with the modeling group (P < 0.01).

b) The effect of cobrotoxin polypeptide on $\alpha$1 microglobulin ($\alpha$1 mg) of Adriamycin induced diabetic nephropathy in rats, compared with the control group, $\alpha$ 1 microglobulin(A1 mg) of the modeling group was increased significantly; compared with the modeling group, the urine $\alpha$ 1 microglobulin of cobrotoxin polypeptide group decreased significantly. * * It indicates that the cobrotoxin polypeptide group is compared with the modeling group (P < 0.01).

c) The effect of cobrotoxin polypeptide on $\beta$ 2 microglobulin ( $\beta$ 2-mg) of Adriamycin induced diabetic nephropathy in rats, compared with the control group, $\beta$ 2 microglobulin ( $\beta$ 2-mg) of the modeling group was increased significantly; compared with the modeling group, the urine $\beta$ 2 microglobulin of cobrotoxin polypeptide group decreased significantly. * * It indicates that the cobrotoxin polypeptide group is compared with the modeling group (P < 0.05).

d) The effect of cobrotoxin polypeptide on transferrin (TRF) of Adriamycin induced diabetic nephropathy in rats, compared with the control group, transferrin (TRF) of the modeling group was increased significantly; compared with the modeling group, the urine transferrin (TRF) of cobrotoxin polypeptide group decreased significantly. * * It indicates that the cobrotoxin polypeptide group is compared with the modeling group (P < 0.05).

e) The effect of cobrotoxin polypeptide on immunoglobulin (IgG) of Adriamycin induced diabetic nephropathy in rats, compared with the control group, immunoglobulin (IgG) of the modeling group was increased significantly; compared with the modeling group, the urine immunoglobulin (IgG) of cobrotoxin polypeptide group decreased significantly. * * It indicates that the cobrotoxin polypeptide group is compared with the modeling group (P < 0.05).

Table-2 SEQ ID No.2 cobrotoxin polypeptide was used for the treatment group

Table-2

| adriamycin 3+2mg/kg | UALB ($\mu$g/ml) | a1-MG ($\mu$g/ml) | B2-MG (ng/ml) | TRF ($\mu$g/ml) | IgG ($\mu$g/ml) |
| --- | --- | --- | --- | --- | --- |
| control group | 0.52 ±0.13 | 0.34 ±0.08 | 3.16 ±1.05 | 0.03 ±0.006 | 0.11 ±0.04 |
| modeling group | 1.57 ±0.19 * | 1.10 ±0.31 ** | 36.08 ±=3.63 * | 0.18 ±0.087 * | 0.53 ±0.22 * |
| cobrotoxin group | 0.82 ±0.21 | 0.74 ±0.14 | 30.01 ±=6.47 | 0.11 ±0.032 | 0.31 ±0.15 |

a) The effect of cobrotoxin polypeptide on the microalbuminuria (UALB) of adriamycin induced diabetic nephropathy in rats, compared with the control group, the microalbuminuria (UAlb) of the modeling group was significantly increased; compared with the model group, the microalbuminuria of cobrotoxin polypeptide group was significantly lower. * * It indicates that the cobrotoxin polypeptide group is compared with the modeling group (P < 0.05).

b) The effect of cobrotoxin polypeptide on $\alpha$ microglobulin (A1 mg) of Adriamycin induced diabetic nephropathy in rats, compared with the control group, $\alpha$ 1 microglobulin (A1 mg) of the modeling group was increased significantly; compared with the modeling group, urine $\alpha$ 1 microglobulin of cobrotoxin polypeptide group decreased significantly. * * It indicates that the cobrotoxin polypeptide group is compared with the modeling group (P < 0.01).

c) The effect of cobrotoxin polypeptide on $\beta$ 2 microglobulin ( $\beta$ 2-mg) of Adriamycin induced diabetic nephropathy in rats, compared with the control group, β2 microglobulin ( β2-mg) of the modeling group was increased significantly; compared with the modeling group, the urine β2 microglobulin of cobrotoxin polypeptide group decreased significantly. * * It indicates that the cobrotoxin polypeptide group is compared with the modeling group (P < 0.05).

d) The effect of cobrotoxin polypeptide on transferrin (TRF) of Adriamycin induced diabetic nephropathy in rats, compared with the control group, transferrin (TRF) of the modeling group was increased significantly; compared with the modeling group, the urine transferrin (TRF) of cobrotoxin polypeptide group decreased significantly. * * It indicates that the cobrotoxin polypeptide group is compared with the modeling group (P < 0.05).

e) The effect of cobrotoxin polypeptide on immunoglobulin (IgG) of Adriamycin induced diabetic nephropathy in rats, compared with the control group, immunoglobulin (IgG) of the modeling group was increased significantly; compared with the modeling group, the urine immunoglobulin (IgG) of cobrotoxin polypeptide group decreased significantly. * * It indicates that the cobrotoxin polypeptide group is compared with the modeling group (P < 0.05).

Example C: Use cobra neurotoxin polypeptides SEQ ID No.1 and SEQ ID No.2 for the treatment of t streptozotocin (STZ) - induced nephritis proteinuria in rat

1 . Experimental animals and groups

The experimental rats were divided into two groups, 30 rats in each group, 10 rats in the treatment group; Modeling group 10; 10 rats in the control group, detail as follows:
80 male SD rats weighing 160-180g were randomly divided into 2 groups: 20 in the control group and 60 in the model group. Forty rats in model group, after success in modeling, were randomly divided into cobrotoxin polypeptide treatment group and model group.

Each group of the three, namely control group, treatment group and model group were randomly divided into two groups again, with 10 rats in each group to test cobrotoxin peptide SEQ ID No. 1 and SEQ ID No. 2, the remaining rats were out of the groups. The treatment group was given cobrotoxin polypeptide • 20ug / kg by gavage once a day, continuously for 8 weeks; the model group and the control group were perfused with normal saline once a day for 8 weeks.

2 . Modeling method

The model rats were fed and fed normally. Each rat was injected intraperitoneally with 0.5ml Freund's complete adjuvant (CFA) and intraperitoneally with streptozotocin (STZ) solution the next day. Before hand, 0.1mmol/l pH4 5 of citric acid buffer was prepared to 1% of the concentration and was injected intraperitoneally at 55mg/kg. After one week, blood glucose was detected by tail vein blood. The blood glucose was maintained above 16.7mmol/L, and the urine glucose 3+ to 4+ was regarded as a successful diabetes modeling.

3. Collection and detection of urinary protein

After the last treatment, the rats were placed in a metabolic cage to collect 10ml of urine, centrifuged at 3500r / min for 10min, and the supernatant was extracted into the cryopreservation tube and kept in the refrigerator at - 80 °C for standby. Urinary a 1-microglobulin β2-microsphere protein, microalbumin, transferrin and immunoglobulin G (IgG), were tested by ELISA immunosorbent assay, operate according to the instructions of ELISA kit.
Anesthetized rats were injected intraperitoneally with 1.5% Pentobarbital Sodium (50mg / kg). After disappearence of corneal reflex, the intraperitoneal cavity was opened, expose the abdominal aorta, collect 10ml of whole blood from the abdominal aorta, place it in a centrifuge tube, stand for 2h, centrifuge at 3500r / min for 10min, and then lift it. Take the supernatant into the cryopreservation tube, store it in the refrigerator at - 80 °C for standby, and use the automatic biochemical analyzer to detect serum creatinine and urine nitrogen, cholesterol and triglyceride.

4. Comparison of urinary protein content between control group, modeling group and treatment group

TABLE-3 SEQ ID No.1 cobrotoxin polypeptide was used for treatment group

Table-3

| streptozotocin (STZ) 55mg/kg | UALB ($\mu$g/ml) | a1-MG ($\mu$g/ml) | B2-MG (ng/ml) | TRF ($\mu$g/ml) | IgG ($\mu$g/ml) |
|---|---|---|---|---|---|
| control group | 0.51 ±0.10 | 0.61 ±0.10 | 10.16±2.31 | 0.05 ±0.02 | 0.13±0.06 |

(continued)

| streptozotocin (STZ) 55mg/kg | UALB (μg/ml) | a1-MG (μg/ml) | B2-MG (ng/ml) | TRF (μg/ml) | IgG (μg/ml) |
|---|---|---|---|---|---|
| modeling | 2.21 ±0.20 ** | 2.11 ‡0.32** | 60.18±15.06 ** | 0.31 ±0.19 * | 0.59±0.28 * |
| cobrotoxin group | 1.21 ±0.14 | 1.02 ±0.21 | 38.90±11.82 | 0.17 ±0.05 | 0.35±0.11 |

a) The effect of cobrotoxin polypeptide on the microalbuminuria (UALB) of streptozotocin (STZ) - induced diabetic nephropathy in rats, compared with the control group, the microalbuminuria (UAlb) of the modeling group was significantly increased; compared with the modeling group, the microalbuminuria of cobrotoxin polypeptide group was significantly lower. * * It indicates that the cobrotoxin polypeptide group is compared with the modeling group (P < 0.01).

b) The effect of cobrotoxin polypeptide on α microglobulin (A1 mg) of streptozotocin (STZ) induced diabetic nephropathy in rats, compared with the control group, α 1 microglobulin of the modeling group was increased significantly; compared with the modeling group, the urine α 1 microglobulin of cobrotoxin polypeptide group decreased significantly. * * It indicates that the cobrotoxin polypeptide group is compared with the modeling group (P < 0.01).

c) The effect of cobrotoxin polypeptide on β 2 microglobulin ( β 2-mg) of streptozotocin (STZ) induced diabetic nephropathy in rats, compared with the control group, β 2 microglobulin ( β 2-mg) of the modeling group was increased significantly; compared with the modeling group, the urine β 2 microglobulin of cobrotoxin polypeptide group decreased significantly. * * It indicates that the cobrotoxin polypeptide group is compared with the modeling group (P < 0.01).

d) The effect of cobrotoxin polypeptide on transferrin (TRF) of streptozotocin (STZ) induced diabetic nephropathy in rats, compared with the control group, transferrin (TRF) increased of the modeling group was significantly; compared with the modeling group, the urine transferrin (TRF) of cobrotoxin polypeptide group decreased significantly. * * It indicates that the cobrotoxin polypeptide group is compared with the modeling group (P < 0.05).

e) The effect of cobrotoxin polypeptide on immunoglobulin (IgG) of streptozotocin (STZ) induced diabetic nephropathy in rats, compared with the control group, immunoglobulin (IgG) of the modeling group was increased significantly; compared with the modeling group, the urine immunoglobulin (IgG) of cobrotoxin polypeptide group decreased significantly. * * It indicates that the cobrotoxin polypeptide group is compared with the modeling group (P < 0.05).

TABLE-4 SEQ ID No.2 cobrotoxin polypeptide was used for treatment group

Table-4

| streptozotocin (STZ) 55mg/kg | UALB (μg/ml) | a1-MG (μg/ml) | B2-MG (μg/ml) | TRF (μg/ml) | IgG (μg/ml) |
|---|---|---|---|---|---|
| control group | 0.38 ±0.12 | 0.53 ±0.12 | 8.52 ±1.80 | 0.07 ±0.01 | 0.11 ±0.04 |
| modeling group | 1.60 ±0.29 ** | 2.27 ±0.23 ** | 45.21 ±11.25 ** | 0.28 ±0.10 * | 0.44 ±0.17 * |
| cobrotoxin group | 0.89 ±0.26 | 0.96 ±0.19 | 30.34 ±9.31 | 0.18 ±0.06 | 0.31 ±0.09 |

a) The effect of cobrotoxin polypeptide on the microalbuminuria (UALB) of streptozotocin (STZ) - induced diabetic nephropathy in rats, compared with the control group, the microalbuminuria (UAlb) of the modeling group was significantly increased; compared with the modeling group, the microalbuminuria of cobrotoxin polypeptide group was significantly lower. * * It indicates that the cobrotoxin polypeptide group is compared with the modeling group (P < 0.01).

b) The effect of cobrotoxin polypeptide on α microglobulin (A1 mg) of streptozotocin (STZ) induced diabetic nephropathy in rats, compared with the control group, α 1 microglobulin of the modeling group was increased significantly; compared with the modeling group, the urine α 1 microglobulin of cobrotoxin polypeptide group decreased significantly. * * It indicates that the cobrotoxin polypeptide group is compared with the modeling group (P < 0.01).

c) The effect of cobrotoxin polypeptide on β 2 microglobulin ( β 2-mg) of streptozotocin (STZ) induced diabetic nephropathy in rats, compared with the control group, β 2 microglobulin ( β 2-mg) of the modeling group was increased significantly; compared with the modeling group, the urine β 2 microglobulin of cobrotoxin polypeptide group decreased significantly. * * It indicates that the cobrotoxin polypeptide group is compared with the modeling group (P < 0.01).

d) The effect of cobrotoxin polypeptide on transferrin (TRF) of streptozotocin (STZ) induced diabetic nephropathy in rats, compared with the control group, transferrin (TRF) of the modeling group was increased significantly; compared with the modeling group, the urine transferrin (TRF) of cobrotoxin polypeptide group decreased significantly. * * It indicates that the cobrotoxin polypeptide group is compared with the modeling group (P < 0.05).

e) The effect of cobrotoxin polypeptide on immunoglobulin (IgG) of streptozotocin (STZ) induced diabetic nephropathy in rats, compared with the control group, immunoglobulin (IgG) of the modeling group was increased significantly; compared with the modeling group, the urine immunoglobulin (IgG) of cobrotoxin polypeptide group decreased significantly. * * It indicates that the cobrotoxin polypeptide group is compared with the modeling group (P < 0.05).

Cobrotoxin polypeptide could also reduce the indexes of blood creatinine, urea nitrogen, cholesterol and triglyceride in rats after modeling, but there was no significant difference between the treatment group and the modeling group (P > 0.05). This may be because in the early stage of nephritis, the indexes of blood creatinine, urea nitrogen, cholesterol and triglyceride change mildly, and the analysis of small samples can not make statistical significance, so the decline of these indexes needs to be confirmed after further expansion of the samples.

References:

Zhang Guiyun. Application of urinary microprotein determination in early diagnosis of nephropathy. Contemporary medicine 2013, 000(027)-146~146

Gao Xuehui. Clinical application of urinary microprotein in early renal injury. Shaanxi JLab Sci) 2001.05 Vol.16 N0.2

3. Song Xue et al;. Clinical observation and diagnostic value of different urinary proteins in renal diseases International Journal of Urology, Volume 32, issue 2, March 2012

4. Zhu Xiaobin. Application value of urine and renal function test in early diagnosis of diabetic nephropathy. Chinese medical guidelines 2017015 (002)-

5. Wang Xiao. The clinical significance of Urinary Microprotein Detection in the diagnosis of diabetic renal damage. Journal of Zhejiang University of traditional Chinese medicine, 2008, 32 (6): 751 ~ 752

6. Su Cainv et al;. The value of blood and urine microalbuminuria in early diagnosis of diabetic nephropathy. Journal of radioimmunology, 2001, 14 (4): 502

7. Duan liping et al;. Analysis of urinary microprotein types and contents in primary nephrotic syndrome and lupus nephropathy in National Tropical Medicine 2003003 (006) - 771 ~ 772

8. Yang Fang et al;. Relationship between changes of Urinary Microprotein and renal damage in reflux nephropathy Journal of Jinan University Natural Science and Medicine Edition 2000021 (004) - 104 ~ 106

9. Shen shunbiao et al;. Clinical significance of urinary microprotein in IgA nephropathy Chinese Journal of Nephrology 1995000 (001) - 34 ~ 34

10. Teng Shoufeng et al;. The value of three microalbuminuria in early diagnosis of diabetic renal injury. Chinese Journal of misdiagnosis 2009009 (034)

11. Shao Aihua et al;. Observation on the results of urinary microprotein in patients with various nephropathy Journal of Zhejiang University of traditional Chinese medicine 2003027 (005) - 31 ~ 32

12. Huo Meifeng. The value of β- 2 microglobulin in the early diagnosis of diabetic nephropathy. Chinese general practice, 2012 (12): 119-120

13. Kang Ping et al;. the significance of β- 2 microglobulin in early diabetic nephropathy. Heilongjiang Medicine 2008

14. Lu Qingyun et al;. Clinical significance of α-1 β-2microglobulin in early renal damage of Primary hypertension [J] Western Traditional Chinese medicine, 2014:131-133

15. Wang Chunwei. the clinical value of urine α-1 microglobulin in diabetic nephropathy. Journal of practical medical technology 2007014 (025)

16. Yan Jingchun. Clinical significance of urine α microglobulin in secondary nephropathy. Chinese Journal of coal industry medicine 2014017 (006)

17. Wei Wenfeng. The significance of urinary Microtransferrin in the early diagnosis of diabetic nephropathy. Labeled immunoassay and clinical, 2002

18. Li Xiaoxia et al;. Observation and comparison of urinary transferrin and albumin in early glomerular injury. Journal of medical forum, 2010

19. Zhu Guangbo et al;. Clinical significance of determination of urinary Microtransferrin in patients with diabetes mellitus. Shaanxi medical journal, 1999

20. Dong Hui et al;. The clinical significance of combined detection of microglobulin and transferrin in the early diagnosis of diabetic nephropathy. Journal of Tianjin Medical University, 2002, 8 (2): 253

21. Martin P, et al;. Urinary transferring and early predictor ofglomerular dysfunction[J].AnnClin Biochem, 1988, 5(Suppl): 158.

22. Liu Qian. Effect of hemoperfusion combined with continuous blood purification on acute renal failure caused by snake bite. Sino foreign medical research 2013000 (036)

23. Zhang qionglin. Nursing experience of continuous renal replacement therapy combined with traditional Chinese medicine hoop therapy in the treatment of severe snake bite. Family psychologist 2014010 (009)

24. Zhang Youqi et al;. Research Progress on kidney injury caused by snake venom toxin. Medical review 2010016 (002)

25. Shengwei Xiong, Chunhong Huang.Synergistic strategies ofpredominant toxins in snake venoms.Toxicology Letters 287(2018)142-154

26. Laustsen, A.H.Toxin synergism in snake venoms.Toxin Reviews, 35(3-4), 165-170.DOI: 10.1080/15569543.2016

27. Anil Kumar, Varun Gupta.Neurological Implications of Dendrotoxin: AReview.EC PHARMACOLOGY AND TOXICOLOGY.May 25, 2018

28. Strydom DJ, Botes DP.Snake venom toxins-I.Preliminary studies onthe separation of toxins of elapidae venoms.Toxicon 8: 203-9.1970

29. Joubert, F.J., Taljaard, N, Snake venoms.The amino-acid sequence ofprotein S2C4 from Dendroaspis jamesoni kaimosae(Jameson's mamba)venom.HoppeSeylers.Z.Physiol.Chem.360, 571-580.1979

30. Weina et al;. Establishment of modified adriamycin nephropathy rat model. Journal of Xi'an Jiaotong University (Medical Edition), 2009, (4): 445-452

31. Li Shifen Streptozotocin induced diabetic nephropathy in SD rats. Chinese Journal of modern medicine 2010-14-007

32. Zhou Guowu et al;. α-7 acetylcholine nicotinic receptors regulate inflammation. Advances in modern biomedicine, issue 14, 2009

33. Liu Zhigang. The regulation of $\alpha$-7 nicotinic acetylcholine receptor and inflammation. China medical guidelines, issue 03, 2014

34.Hong Wang et al; .Nicotinic acetylcholine receptor a7 subunit is an essential regulator of inflammation NATURE|VOL 421|23JANUARY 2003

35.Stéphanie St-Pierre et al; .Nicotinic Acetylcholine Receptors Modulate Bone Marrow-Derived Pro-Inflammatory Monocyte Production and Survival.PLOS ONE DOI: 10.1371/journal.pone.0150230February 29, 2016

**Claims**

1. A method for treating nephropathy proteinuria in a mammal. Said method comprising administering to a mammal in need thereof a pharmaceutical composition of a therapeutically effective amount of elapidae neurotoxin, and a pharmaceutically acceptable carrier base for use in inhibiting or controlling nephropathy proteinuria.

2. According to claims (1), wherein the nephropathy proteinuria is **characterized in that** they are selected from the group consisting of albumin, immunoglobulins, $\beta$ 2 microglobulin, $\alpha$ 1 microglobulin and transferrin, and wherein the urinary protein is over the normal level of medical diagnosis.

3. According to claim (1), wherein the nephropathy proteinuria is further **characterized in that** it comprises the increase of one or multiple, or all levels of urinary protein biomarkers, including albumin, immunoglobulins, $\beta$ 2 microglobulin, $\alpha$ 1 microglobulin and transferrin.

4. The elapidae neurotoxin of claims (1), wherein it is an elapidae neurotoxin polypeptide having the amino acid sequence shown in SEQ ID No.1 to SEQ ID No.14 or elapidae neurotoxin polypeptide homologues having 70% or more homology with the elapidae neurotoxin polypeptide of SEQ ID No.1 to SEQ ID No.14 and the biological function of the elapidae neurotoxin polypeptide homologues is the same as or similar to that of the elapidae neurotoxin polypeptide of the amino acid sequence ID No. 1 to SEQ ID No. 14.

5. Elapidae neurotoxin polypeptides or elapidae neurotoxin polypeptides homologues according to claims (1), are further **characterized in that** they are derived from natural snake venoms, or synthesized from chemical polypeptides, or obtained from prokaryotic or eukaryotic hosts using recombinant technology (such as Bacteria, yeast, higher plants, insects and mammalian cells).

6. The recombinantly produced elapidae neurotoxin polypeptide or its homologues according to claim (5), based on the host used in the recombinant production scheme, the polypeptide or its homologues of the present invention may be glycosylated, or may be non-glycosylated; Disulfide-bonded or non-disulfide-bonded. The polypeptides and its homologues described in the present invention may also include or exclude the starting methionine residue.

7. The elapidae neurotoxin polypeptide as in any of claims (1,4,5,6,), further **characterized in that** the polypeptide in the present invention may include fragments of the above-mentioned various elapidae neurotoxin polypeptides after hydrolysis or enzymolysis, derivatives or analogs treated by physical, chemical or biological method, they are polypeptides which basically maintain the same biological function or activity as the above-mentioned elapidae neurotoxin polypeptide. The fragments, derivatives or analogs described in the present invention may be a polypeptide in which one or more amino acid residues are substituted, or a polypeptide having a substituent group in one or more amino acid residues, or combined with a compound (such as compounds that extend the half-life of a polypeptide, such as polyethylene glycol), or a polypeptide formed by fusion of a fatty chain, or a polypeptide formed by fusing an additional amino acid sequence to this polypeptide sequence. As described herein, these fragments, derivatives, and analogs are within the scope of those skilled in the art.

8. The method of claims (1) comprising intravenous, intramuscular, subcutaneous, intra-articular, oral, sublingual, nasal, rectal, topical, intradermal, intraperitoneal, intrathecal administration or transdermal administration.

9. The dose of elapidae neurotoxin of the method of claim (1) includes from 1 $\mu$g / Kg to 350 $\mu$g / kg each time, and the injection frequency ranges from once a day to multiple times a day, or multiple times a year.

Fig 1

## INTERNATIONAL SEARCH REPORT

| International application No. |
|---|
| **PCT/CN2020/000197** |

| **A.** | **CLASSIFICATION OF SUBJECT MATTER** |
|---|---|

A61K 38/17(2006.01)i;  A61P 13/12(2006.01)i

According to International Patent Classification (IPC) or to both national classification and IPC

| **B.** | **FIELDS SEARCHED** |
|---|---|

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, CNKI, 百度学术搜索, ISI-WEB OF SCIENCE, PUBMED: 蛇, 神经毒素, 蛋白尿, SNAKE, NEUROTOXIN, PROTEINURIA Genbank or EMBL, 中国专利生物序列检索系统: sequence search for SEQ ID NO: 1

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| PX | CN 110755603 A (QI, Zhankai) 07 February 2020 (2020-02-07) 1-9 | 1-9 (all in part) |
| A | CN 102846666 A (SUZHOU RENBEN PHARMACEUTICAL CO., LTD.) 02 January 2013 (2013-01-02) see entire document | 1-9 (all in part) |
| A | CN 102526695 A (SUZHOU RENBEN PHARMACEUTICAL CO., LTD.) 04 July 2012 (2012-07-04) see entire document | 1-9 (all in part) |
| A | US 2009209468 A1 (REID PAUL F et al.) 20 August 2009 (2009-08-20) see entire document | 1-9 (all in part) |
| Y | 王姝之 (WANG, Shuzhi). "中华眼镜蛇毒及神经毒素通过抑制炎症对急慢性肾病发挥保护作用 (Naja Naja Atra Venom And Cobrotoxin Ameliorate Acute And Chronic Nephropathy through Inhibiting Inflammation)" 中国博士学位论文全文数据库, 医药卫生科技辑 (Chinese Doctoral Dissertations Full-text Database, Medicine & Public Health), 15 November 2016 (2016-11-15), E067-1, see page 34 | 1-9 (all in part) |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 November 2020** | **02 December 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2020/000197**

### C. DOCUMENTS CONSIDERED TO BE RELEVANT

| Category* | Citation of document, with indication, where appropriate, of the relevant passages | Relevant to claim No. |
|---|---|---|
| Y | "ACCESSION NO: P60770"<br>*Genbank Database,* 12 November 2017 (2017-11-12),<br>see sequence and related information thereof | 1-9 (all in part) |
| A | Yin-Li Xu et al. "Neurotoxin from Naja naja atra venom inhibits skin allograft rejection in rats"<br>*International Immunopharmacology,* Vol. 28, 10 June 2015 (2015-06-10),<br>ISSN: 1567-5769,<br>pp. 188-198, see entire document | 1-9 (all in part) |

Form PCT/ISA/210 (second sheet) (January 2015)

## INTERNATIONAL SEARCH REPORT

| International application No. |
| --- |
| **PCT/CN2020/000197** |

**Box No. II       Observations where certain claims were found unsearchable (Continuation of item 2 of first sheet)**

This international search report has not been established in respect of certain claims under Article 17(2)(a) for the following reasons:

1. ☑   Claims Nos.: **1-9**
   because they relate to subject matter not required to be searched by this Authority, namely:

   [1]   The subject matter of claims 1-9 relates to a method for treatment of the human or animal body, and thus belongs to subject matter which does not require a search as stipulated in PCT Rule 39.1(iv). The following search and opinion for claims 1-9 are provided on the basis of an expectation that the subject matter of claims 1-9 should be "a use of a composition in preparing a drug for treating proteinuria in a patient, the composition being a composition containing a therapeutically effective dose of a cobra neurotoxin polypeptide and a pharmaceutically acceptable carrier."

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

**Box No. III       Observations where unity of invention is lacking (Continuation of item 3 of first sheet)**

This International Searching Authority found multiple inventions in this international application, as follows:

[1]   Invention 1: claims 1-9 (all in part), relating to the subject matter related to the amino acid sequence shown in SEQ ID NO: 1;

[2]   Inventions 2-4: claims 1-9 (all in part), respectively relating to the subject matter related to any amino acid sequence in SEQ ID NOs: 2-14.

[3]   Because SEQ ID NOs: 1-14 do not share a common structure, their common feature is merely that they are all cobra neurotoxins. Therefore, the common technical feature of inventions 1-14 is: using a composition containing a cobra neurotoxin to control proteinuria. A prior art document (Wang Shuzhi, Naja Naja Atra Venom And Cobrotoxin Ameliorate Acute And Chronic Nephropathy through Inhibiting Inflammation, Chinese Doctoral Dissertations Full-text Database, Medicine & Public Health, November 2016, E067-1, see page 34), discloses that cobra neurotoxins can lessen proteinuria, and thus it is apparent that the common technical feature of inventions 1-14 is already disclosed in the prior art, and cannot serve as a special technical feature. Therefore, inventions 1-14 do not share a same or corresponding special technical feature, do not form a single general inventive concept, lack unity, and do not comply with PCT Rules 13.1, 13.2, and 13.3.

Form PCT/ISA/210 (continuation of first sheet) (January 2015)

**INTERNATIONAL SEARCH REPORT**

| International application No. |
| --- |
| **PCT/CN2020/000197** |

**Box No. III    Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-9 (all in part), relate to the subject matter related to the amino acid sequence shown in SEQ ID NO: 1.**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2020/000197**

| Patent document cited in search report | | | Publication date (day/month/year) | Patent family member(s) | | | Publication date (day/month/year) |
|---|---|---|---|---|---|---|---|
| CN | 110755603 | A | 07 February 2020 | None | | | |
| CN | 102846666 | A | 02 January 2013 | CN | 102846666 | B | 18 November 2015 |
| CN | 102526695 | A | 04 July 2012 | None | | | |
| US | 2009209468 | A1 | 20 August 2009 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201210228609

### Non-patent literature cited in the description

- **SONG XUE et al.** Clinical observation and diagnostic value of different urinary proteins. *International Journal of Urology,* March 2012, vol. 32 (2
- **ZHU XIAOBIN.** Application value of urine and renal function test in early diagnosis of diabetic nephropathy. *Chinese medical guidelines*
- **WANG XIAO.** The clinical significance of Urinary Microprotein Detection in the diagnosis of diabetic renal damage. *Journal of Zhejiang University of traditional Chinese medicine,* 2008, vol. 32 (6), 751-752
- **SU CAINV et al.** The value of blood and urine microalbuminuria in early diagnosis of diabetic nephropathy. *Journal of radioimmunology,* 2001, vol. 14 (4), 502
- **DUAN LIPING et al.** Analysis of urinary microprotein types and contents in primary nephrotic syndrome and lupus nephropathy. *National Tropical Medicine*
- **YANG FANG et al.** Relationship between changes of Urinary Microprotein and renal damage in reflux nephropathy. *Journal of Jinan University Natural Science and Medicine*
- **SHEN SHUNBIAO et al.** Clinical significance of urinary microprotein in IgA nephropathy. *Chinese Journal of Nephrology*
- **TENG SHOUFENG et al.** The value of three microalbuminuria in early diagnosis of diabetic renal injury. *Chinese Journal of misdiagnosis*
- **SHAO AIHUA et al.** Observation on the results of urinary microprotein in patients with various nephropathy. *Journal of Zhejiang University of traditional Chinese medicine*
- **HUO MEIFENG.** The value of β- 2 microglobulin in the early diagnosis of diabetic nephropathy. *Chinese general practice,* 2012, (12), 119-120
- **KANG PING et al.** the significance of β- 2 microglobulin in early diabetic nephropathy. *Heilongjiang Medicine,* 2008
- **LU QINGYUN et al.** Clinical significance of α-1 β-2microglobulin in early renal damage of Primary hypertension. *Western Traditional Chinese medicine,* 2014, 131-133
- **WANG CHUNWEI.** the clinical value of urine α-1 microglobulin in diabetic nephropathy. *Journal of practical medical technology*
- **YAN JINGCHUN.** Clinical significance of urine α microglobulin in secondary nephropathy. *Chinese Journal of coal industry medicine*
- **WEI WENFENG.** The significance of urinary Microtransferrin in the early diagnosis of diabetic nephropathy. *Labeled immunoassay and clinical,* 2002
- **LI XIAOXIA et al.** Observation and comparison of urinary transferrin and albumin in early glomerular injury. *Journal of medical forum,* 2010
- **ZHU GUANGBO et al.** Clinical significance of determination of urinary Microtransferrin in patients with diabetes mellitus. *Shaanxi medical journal,* 1999
- **DONG HUI et al.** The clinical significance of combined detection of microglobulin and transferrin in the early diagnosis of diabetic nephropathy. *Journal of Tianjin Medical University,* 2002, vol. 8 (2), 253
- **MARTIN P et al.** Urinary transferring and early predictor ofglomerular dysfunction[J. *AnnClin Biochem,* 1988, vol. 5 (158
- **LIU QIAN.** Effect of hemoperfusion combined with continuous blood purification on acute renal failure caused by snake bite. *Sino foreign medical research*
- **ZHANG QIONGLIN.** Nursing experience of continuous renal replacement therapy combined with traditional Chinese medicine hoop therapy in the treatment of severe snake bite. *Family psychologist*
- **ZHANG YOUQI et al.** Research Progress on kidney injury caused by snake venom toxin. *Medical review*
- **SHENGWEI XIONG.** Chunhong Huang.Synergistic strategies ofpredominant toxins in snake venoms. *Toxicology Letters,* 2018, vol. 287, 142-154
- **LAUSTSEN, A.H.** Toxin synergism in snake venoms. *Toxin Reviews,* vol. 35 (3-4
- **ANIL KUMAR ; VARUN GUPTA.** Neurological Implications of Dendrotoxin: AReview. *EC PHARMACOLOGY AND TOXICOLOGY,* 25 May 2018

- **STRYDOM DJ ; BOTES DP.** Snake venom toxins-I.Preliminary studies onthe separation of toxins of elapidae venoms. *Toxicon,* 1970, vol. 8, 203-9
- **JOUBERT, F.J. ; TALJAARD, N.** Snake venoms.The amino-acid sequence ofprotein S2C4 from Dendroaspis jamesoni kaimosae(Jameson's mamba)venom. *HoppeSeylers.Z.Physiol.Chem.,* 1979, vol. 360, 571-580
- **WEINA et al.** Establishment of modified adriamycin nephropathy rat model. *Journal of Xi'an Jiaotong University (Medical Edition),* 2009, (4), 445-452
- **LI SHIFEN.** Streptozotocin induced diabetic nephropathy in SD rats. *Chinese Journal of modern medicine*
- **ZHOU GUOWU et al.** $\alpha$-7 acetylcholine nicotinic receptors regulate inflammation. *Advances in modern biomedicine,* 2009, (14
- **LIU ZHIGANG.** The regulation of $\alpha$-7 nicotinic acetylcholine receptor and inflammation. *China medical guidelines,* 2014, (03
- **HONG WANG et al.** Nicotinic acetylcholine receptor a7 subunit is an essential regulator of inflammation. *NATURE,* 23 January 2003, vol. 421
- **STÉPHANIE ST-PIERRE et al.** *Nicotinic Acetylcholine Receptors Modulate Bone Marrow-Derived Pro-Inflammatory Monocyte Production and Survival,* 29 February 2016

SEQUENCE LISTING

<110>

<120>

<130>  PCT-003

<160>  14

<170>  PatentIn version 3.5

<210>  1
<211>  62
<212>  PRT
<213>  Naja atra or Naja kaouthia

<400>  1

```
Leu Glu Cys His Asn Gln Gln Ser Ser Gln Thr Pro Thr Thr Thr Gly
1               5                   10                  15

Cys Ser Gly Gly Glu Thr Asn Cys Tyr Lys Lys Arg Trp Arg Asp His
            20                  25                  30

Arg Gly Tyr Arg Thr Glu Arg Gly Cys Gly Cys Pro Ser Val Lys Asn
        35                  40                  45

Gly Ile Glu Ile Asn Cys Cys Thr Thr Asp Arg Cys Asn Asn
        50                  55                  60
```

<210>  2
<211>  83
<212>  PRT
<213>  Naja atra or Naja kaouthia

<400>  2

```
Met Lys Thr Leu Leu Leu Thr Leu Leu Val Val Thr Ile Val Cys Leu
1               5                   10                  15

Asp Leu Gly Tyr Thr Leu Glu Cys His Asn Gln Gln Ser Ser Gln Thr
            20                  25                  30

Pro Thr Thr Thr Gly Cys Ser Gly Gly Glu Thr Asn Cys Tyr Lys Lys
        35                  40                  45

Arg Trp Arg Asp His Arg Gly Tyr Arg Thr Glu Arg Gly Cys Gly Cys
        50                  55                  60

Pro Ser Val Lys Asn Gly Ile Glu Ile Asn Cys Cys Thr Thr Asp Arg
65                  70                  75                  80

Cys Asn Asn
```

```
<210>  3
<211>  62
<212>  PRT
<213>  Naja atra or Naja kaouthia

<400>  3

Leu Glu Cys His Asn Gln Gln Ser Ser Gln Thr Pro Thr Thr Thr Gly
1               5                   10                  15

Cys Ser Gly Gly Glu Thr Asn Cys Tyr Lys Lys Arg Trp Arg Asp His
            20                  25                  30

Arg Gly Tyr Arg Thr Glu Arg Gly Cys Gly Cys Pro Ile Val Lys Asn
            35                  40                  45

Gly Ile Glu Ser Asn Cys Cys Thr Thr Asp Arg Cys Asn Asn
        50                  55                  60


<210>  4
<211>  74
<212>  PRT
<213>  Bungarus multicinctus

<400>  4

Ile Val Cys His Thr Thr Ala Thr Ser Pro Ile Ser Ala Val Thr Cys
1               5                   10                  15

Pro Pro Gly Glu Asn Leu Cys Tyr Arg Lys Met Trp Cys Asp Ala Phe
            20                  25                  30

Cys Ser Ser Arg Gly Lys Val Val Glu Leu Gly Cys Ala Ala Thr Cys
            35                  40                  45

Pro Ser Lys Lys Pro Tyr Glu Glu Val Thr Cys Cys Ser Thr Asp Lys
        50                  55                  60

Cys Asn Pro His Pro Lys Gln Arg Pro Gly
65                  70


<210>  5
<211>  74
<212>  PRT
<213>  Bungarus multicinctus

<400>  5

Ile Val Cys His Thr Thr Ala Thr Ile Pro Ser Ser Ala Val Thr Cys
1               5                   10                  15
```

```
Pro Pro Gly Glu Asn Leu Cys Tyr Arg Lys Met Trp Cys Asp Ala Phe
        20                  25                  30

Cys Ser Ser Arg Gly Lys Val Val Glu Leu Gly Cys Ala Ala Thr Cys
        35                  40                  45

Pro Ser Lys Lys Pro Tyr Glu Glu Val Thr Cys Cys Ser Thr Asp Lys
    50                  55                  60

Cys Asn His Pro Pro Lys Arg Gln Pro Gly
65                  70
```

```
<210>   6
<211>   72
<212>   PRT
<213>   Ophiophagus hannah

<400>   6
```

```
Ile Arg Cys Phe Ile Thr Pro Asp Val Thr Ser Gln Ala Cys Pro Asp
1               5                   10                  15

Gly Gln Asn Ile Cys Tyr Thr Lys Thr Trp Cys Asp Asn Phe Cys Gly
        20                  25                  30

Met Arg Gly Lys Arg Val Asp Leu Gly Cys Ala Ala Thr Cys Pro Thr
        35                  40                  45

Val Lys Pro Gly Val Asp Ile Lys Cys Cys Ser Thr Asp Asn Cys Asn
    50                  55                  60

Pro Phe Pro Thr Arg Glu Arg Ser
65                  70
```

```
<210>   7
<211>   71
<212>   PRT
<213>   Ophiophagus hannah

<400>   7
```

```
Ile Arg Cys Phe Ile Thr Pro Asp Val Thr Ser Gln Ala Cys Pro Asp
1               5                   10                  15

Gly His Val Cys Tyr Thr Lys Met Trp Cys Asp Asn Phe Cys Gly Met
        20                  25                  30

Arg Gly Lys Arg Val Asp Leu Gly Cys Ala Ala Thr Cys Pro Lys Val
        35                  40                  45
```

15

Lys Pro Gly Val Asn Ile Lys Cys Cys Ser Arg Asp Asn Cys Asn Pro
    50                  55                  60

Phe Pro Thr Arg Lys Arg Ser
65                  70


<210>   8
<211>   72
<212>   PRT
<213>   Ophiophagus hannah

<400>   8

Thr Lys Cys Tyr Lys Thr Gly Asp Arg Ile Ile Ser Glu Ala Cys Pro
1               5               10                  15

Pro Gly Gln Asp Leu Cys Tyr Met Lys Thr Trp Cys Asp Val Phe Cys
            20                  25                  30

Gly Thr Arg Gly Arg Val Ile Glu Leu Gly Cys Thr Ala Thr Cys Pro
            35                  40                  45

Thr Val Lys Pro His Glu Gln Ile Thr Cys Cys Ser Thr Asp Asn Cys
    50                  55                  60

Asp Pro His His Lys Met Leu Gln
65                  70


<210>   9
<211>   61
<212>   PRT
<213>   Naja kaouthia

<400>   9

Leu Glu Cys His Asn Gln Gln Ser Ser Gln Ala Pro Thr Thr Lys Thr
1               5               10                  15

Cys Ser Gly Glu Thr Asn Cys Tyr Lys Lys Trp Trp Ser Asp His Arg
            20                  25                  30

Gly Thr Ile Ile Glu Arg Gly Cys Gly Cys Pro Lys Val Lys Pro Gly
            35                  40                  45

Val Asn Leu Asn Cys Cys Arg Thr Asp Arg Cys Asn Asn
    50                  55                  60


<210>   10
<211>   62
<212>   PRT
<213>   Naja kaouthia

<400> 10

Leu Glu Cys His Asn Gln Gln Ser Ile Gln Thr Pro Thr Thr Thr Gly
1               5                   10                  15

Cys Ser Gly Gly Glu Thr Asn Cys Tyr Lys Lys Arg Trp Arg Asp His
            20                  25                  30

Arg Gly Tyr Arg Thr Glu Arg Gly Cys Gly Cys Pro Ser Val Lys Asn
            35                  40                  45

Gly Ile Glu Ile Asn Cys Cys Thr Thr Asp Arg Cys Asn Asn
    50                  55                  60

<210> 11
<211> 83
<212> PRT
<213> Naja kaouthia

<400> 11

Met Lys Thr Leu Leu Leu Thr Leu Leu Val Val Thr Ile Val Cys Leu
1               5                   10                  15

Asp Leu Gly Tyr Thr Leu Glu Cys His Asn Gln Gln Ser Ser Gln Thr
            20                  25                  30

Pro Thr Thr Thr Gly Cys Ser Gly Gly Glu Thr Asn Cys Tyr Lys Lys
            35                  40                  45

Arg Trp Arg Asp His Arg Gly Tyr Arg Thr Glu Arg Gly Cys Gly Cys
        50                  55                  60

Pro Ser Val Lys Asn Gly Ile Glu Ile Asn Cys Cys Thr Thr Asp Arg
65                  70                  75                  80

Cys Asn Asn

<210> 12
<211> 60
<212> PRT
<213> Dendroaspis polylepis

<220>
<221> misc_feature
<222> (1)..(1)
<223> Xaa can be any naturally occurring amino acid

<400> 12

Xaa Ile Cys Tyr Asn His Gln Ser Thr Thr Arg Ala Thr Thr Lys Ser

```
              1                    5                        10                         15

              Cys Glu Glu Asn Ser Cys Tyr Lys Lys Tyr Trp Arg Asp His Arg Gly
                          20                  25                  30

              Thr Ile Ile Glu Arg Gly Cys Gly Cys Pro Lys Val Lys Pro Gly Val
                          35                  40                  45

              Gly Ile His Cys Cys Gln Ser Asp Lys Cys Asn Tyr
                          50                  55                  60


              <210>  13
              <211>  60
              <212>  PRT
              <213>  Dendroaspis polylepis

              <400>  13

              Arg Ile Cys Tyr Asn His Gln Ser Thr Thr Arg Ala Thr Thr Lys Ser
              1                    5                        10                         15

              Cys Glu Glu Asn Ser Cys Tyr Lys Lys Tyr Trp Arg Asp His Arg Gly
                          20                  25                  30

              Thr Ile Ile Glu Arg Gly Cys Gly Cys Pro Lys Val Lys Pro Gly Val
                          35                  40                  45

              Gly Ile His Cys Cys Gln Ser Asp Lys Cys Asn Tyr
                          50                  55                  60


              <210>  14
              <211>  72
              <212>  PRT
              <213>  Dendroaspis polylepis

              <400>  14

              Arg Thr Cys Asn Lys Thr Phe Ser Asp Gln Ser Lys Ile Cys Pro Pro
              1                    5                        10                         15

              Gly Glu Asn Ile Cys Tyr Thr Lys Thr Trp Cys Asp Ala Trp Cys Ser
                          20                  25                  30

              Arg Arg Gly Lys Ile Val Glu Leu Gly Cys Ala Ala Thr Cys Pro Lys
                          35                  40                  45

              Val Lys Ala Gly Val Gly Ile Lys Cys Cys Ser Thr Asp Asn Cys Asn
                          50                  55                  60

              Leu Phe Lys Phe Gly Lys Pro Arg
              65                  70
```

18